# EUROPEAN PATENT SPECIFICATION

(11) **EP 0 447 421 B1**
(45) Date of publication and mention of the grant of the patent: **15.12.1993**
(21) Application number: 89913255.9
(22) Date of filing: 17.11.1989
(51) Int. Cl.: C07C 309/89, C07C 313/04, C07C 315/00, C07C 317/44

(54) **PREPARATION OF 2-(CHLORO, BROMO OR NITRO)-4-(ALKYLSULFONYL)BENZOIC ACIDS AND INTERMEDIATES**
HERSTELLUNG VON 2-(CHLORO, BROMO ODER NITRO)-4-(ALKYLSULFONYL)-BENZOESÄURE UND ZWISCHENPRODUKTE
PREPARATION D'ACIDES 2-(CHLORO, BROMO OU NITRO)-4-(ALKYL-SULFONYL)BENZOIQUES ET LEURS INTERMEDIAIRES

(30) Priority: 07.12.1988 US 280787
(43) Date of publication of application: 25.09.1991
(73) Proprietor: ZENECA INC., Wilmington, Delaware 19897 (US)
(72) Inventor: BROWN, Richard, W., Richmond, CA 94805 (US)
(74) Representative: Greaves, Carol Pauline
(86) International application number: US8905165
(87) International publication number: WO9006301

(56) References cited:
- US-A- 4 211 723

## Description

This invention relates to a process for the preparation of 2-(chloro, bromo or nitro)-4-(alkylsulfonyl)benzoic acids and to intermediates useful in the process.

These 2-(chloro, bromo or nitro)-4-(alkylsulfonyl)benzoic acids are intermediates useful for the preparation of certain 2-(2-chloro, bromo or nitro)-4-(alkylsulfonyl)benzoyl-1,3-cyclohexanedione herbicides. The 2-(chloro, bromo or nitro)-4-(alkylsulfonyl)benzoic acid is converted to its acid chloride or cyanide and it is reacted with certain 1,3-cyclohexanediones according to the process of U.S. Patent 4,695,673 or U.S. Pat. 4,708,127.

### Summary of the Invention

One embodiment of this invention is directed to a process for the preparation of 2-(chloro, bromo or nitro)-4- (alkylsulfonyl)benzoic acids, represented by the following reaction steps:

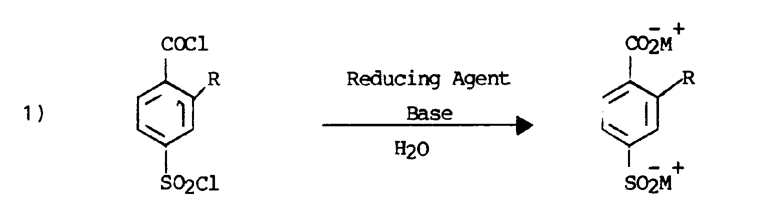

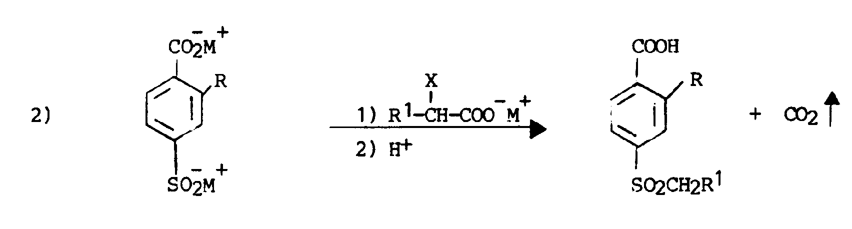

The 2-(chloro, bromo or nitro)-4-(chlorosulfonyl)benzoyl chloride [reactant of Step (1)] can be prepared by the following reaction:

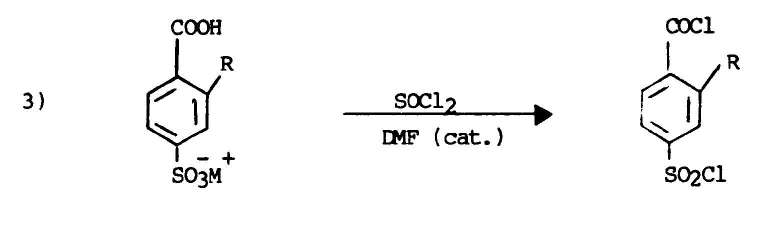

In the above Reaction Steps (1), (2) and (3), R is chloro, bromo or nitro; R¹ is hydrogen or C₁-C₃ alkyl, preferably methyl or ethyl, most preferably R¹ is hydrogen; M is hydrogen, sodium, potassium or ammonium, preferably sodium; and X is chloro, bromo or iodo, preferably chloro.

A second embodiment of this invention is the intermediate reaction product of Reaction Step (1). This intermediate has the structural formula 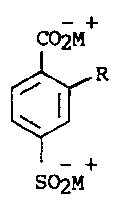
wherein R is chloro, bromo or nitro and M is hydrogen, sodium, potassium or ammonium.

### Detailed Description of the Invention

Referring to the three reaction steps under the "Summary of the Invention" section, the following is additionally taught.

The process of this invention is depicted by Reaction Steps (1) and (2). Step (3) is provided to show a process for the preparation of the intermediate 2-(chloro, bromo or nitro)-4-(chlorosulfonyl)benzoyl chloride. Also the intermediate reaction product of Reaction Step (1) is an embodiment of the invention.

Generally in Reaction Step (1) the 2-(chloro, bramo or nitro)-4-(chlorosulfonyl)benzoyl chloride reaction product of Reaction Step (3) preferably is reacted with an equimolar amount of a reducing agent, preferably sodium or potassium sulfite, along with a base, preferably an inorganic base, having a sodium, potassium or ammonium cation. Such bases are sodium or potassium hydroxide, sodium or potassium bicarbonate or ammonium hydroxide. Preferably the base is sodium bicarbonate. Generally about 3 or more moles of the base are used, preferably between about 4.0 to 4.5 moles. The reaction preferably should be run at a pH of about 7-9. The reaction is run in water, optionally in the presence of a non-miscible organic solvent. During Reaction Step (1), the sulfonyl chloride group is reduced to the sodium, potassium or ammonium salt of the corresponding sulfinate and the acid chloride group is hydrolyzed to the sodium, potassium or ammonium salt of the benzoic acid. Reaction Step (1) is preferably run at a temperature above about 0°C, more preferably between about 20°C to about 100°C, even more preferably between about 30°C to about 75°C and most preferably between about 40°C and about 50°C. When "R" is nitro, the reaction preferably should be run at a temperature between about 10°C to about 20°C. The reaction at the most preferable temperature or above is very quick and normally takes less than 15 minutes.

The reaction product is soluble in the aqueous solvent and normally is not isolated.

Reaction Step (2) is carried out by adding a mole of the sodium, potassium or ammonium salt of an α-halocarboxylic acid of the structural formula 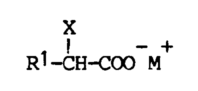
wherein R¹ is hydrogen, C₁-C₃ alkyl, preferably methyl, ethyl or n-propyl, more preferably hydrogen; M is sodium, potassium or ammonium; and X is chloro, bromo or iodo, preferably chloro, to the aqueous reaction product of Reaction Step (1). In the alternative, the above salt of an α-halocarboxylic acid can be formed in situ by adding equal molar amounts of an α-halocarboxylic acid of the structural formula 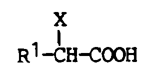
where R¹ and X are as defined with an inorganic base having a sodium, potassium or ammonium cation. Such bases are the same as defined in Reaction Step (1). The reaction is carried to completion by heating the reaction mixture to reflux for about 6-48 hours. The reaction time can be reduced by heating the reaction mixture at higher temperatures under pressure.

Generally in Reaction Step (3), the reaction between the benzoic acid reactant and the thionyl chloride is run in conventional equipment using excess thionyl chloride and at least 5 mole percent of the dimethylformamide (DMF) catalyst. Preferably, the reaction is run in an inert solvent such as toluene or ethylene dichloride, or excess thionyl chloride can serve as the solvent. Preferably, the reaction is run at a temperature between about 50°C to about 100°C in the absence of moisture. The reaction product is recovered by conventional techniques such as separating solids by filtration and evaporating excess solvent from the filtrate to yield the desired reaction product.

As previously recited, an embodiment of this invention is directed to a process for preparing 2,4-disubstituted benzoic acid compounds having the structural formula 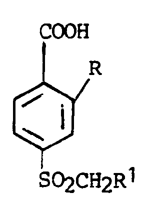
wherein R is chloro, bromo or nitro and R¹ is hydrogen or C₁-C₃ alkyl, by
1) reacting a compound having the structural formula 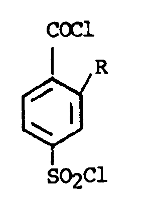 wherein R is as defined with a reducing agent along with a base, preferably an inorganic base, having a sodium, potassium or ammonium cation in an aqueous medium to prepare an intermediate having the structural formula 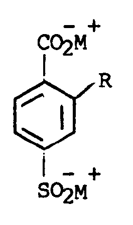 wherein R is chloro, bromo or nitro and M is hydrogen, sodium, potassium or ammonium, followed by
2) reacting the intermediate from step (1) with a salt having the structural formula 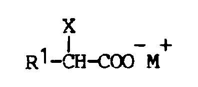 wherein X is chloro, bromo or iodo; R¹ is hydrogen or C₁-C₃ alkyl and M is sodium, potassium or ammonium, to prepare a salt of a benzoic acid having the structural formula 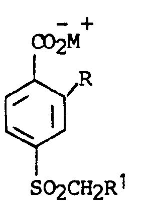 wherein M, R and R¹ are as defined and finally acidifying the salt to prepare the desired product having the structural formula 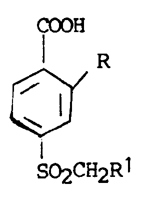 wherein R and R¹ are as defined.

The intermediate benzoic acids of Reaction Step (2) can easily be converted to their respective acid chlorides and then to their acid cyanides, if desired, by the following two reactions. First, a mole of oxalyl chloride and a catalytic amount of dimethylformamide in a suitable solvent such as methylene chloride is heated at a temperature of 20 to 40°C for 1 to 4 hours with a mole of the intermediate benzoic acid. The corresponding benzoic acid cyanide can easily be prepared from the benzoic acid chloride by reaction with cuprous cyanide at a temperature of 50 to 220°C for 1 to 2 hours.

The following series of examples teach the synthesis of representative intermediate compounds of this invention and the processes of this invention. The structures of all compounds of the examples and tables were verified by nuclear magnetic resonance (NMR), infrared spectroscopy (IR) and mass spectroscopy (MS).

### EXAMPLE 1

### 2-Chloro-4-(chlorosulfonyl)benzoyl Chloride

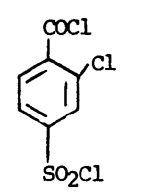

A 25 milliliter (mL) round bottom flask equipped with reflux condenser, thermometer, and magnetic stirrer was charged with 3.2 grams (g) [10.0 millimole (mmol)] of the potassium salt of 2-chloro-4-sulfobenzoic acid (85 wt. % pure), 10 mL (140 mmol) of thionyl chloride and 0.03 g (0.5 mmol) of dimethylformamide (DMF). After heating for one hour at 60°C, the cooled reaction mixture was diluted with 10 mL of toluene, filtered and evaporated to afford 2.55 g (100% yield) of 2-chloro-4-(chlorosulfonyl)benzoyl chloride as a brown oil.

### EXAMPLE 2

### 2-Chloro-4-(methylsulfonyl)benzoic Acid Using Chloroacetic Acid and Sodium Hydroxide

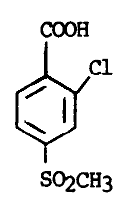

A 250 mL round bottom flask equipped with a reflux condenser, thermometer, and magnetic stirrer was charged with 9.2 g (73 mmol) of sodium sulfite, 24.6 g (292 mmol) of sodium bicarbonate and 80 mL of water. The resulting slurry was heated to 50°C, and 20.0 g (70 mmol) of 2-chloro-4-(chlorosulfonyl)benzoic acid was added over 15 minutes. After heating at 50°C for three hours, chloroacetic acid (10.4 g, 110 mmol) and 5.7 mL (110 mmol) of 50% aqueous sodium hydroxide were added sequentially to the aqueous solution of 2-chloro-4-sulfinylbenzoic acid, and the reaction mixture heated to reflux. After heating for 19 hours, the reaction mixture was allowed to cool to ambient temperature and acidified with dilute HCl. The precipitated solids were collected by filtration, washed with dilute HCl and dried to give 17.4 g of 2-chloro-4-(methylsulfonyl)benzoic acid as a white solid. The solid was assayed at 85% purity, corresponding to an overall yield of 89%.

### EXAMPLE 3

### 2-Chloro-4-(methylsulfonyl)benzoic Acid Using the Sodium Salt of Chloroacetic Acid

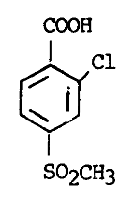

A 100 mL round bottom flask equipped with a reflux condenser, thermometer, and magnetic stirrer was charged with 4.6 g (37 mmol) of sodium sulfite, 12.3 g (146 mmol) of sodium bicarbonate and 40 mL of water. The resulting slurry was heated to 75°C and 10 g (33 mmol) of 2-chloro-4-(chlorosulfonyl)benzoyl chloride was added slowly. After stirring at 75°C for 2 hours, 6.4 g (55 mmol) of the sodium salt of chloroacetic acid was added and the reaction mixture heated at reflux for 21 hours. The cooled reaction mixture was acidified with dilute HCl and extracted with ethyl acetate. The organic layer was dried with magnesium sulfate and evaporated to dryness to afford 7.5 g of 2-chloro-4-(methylsulfonyl)benzoic acid as a white solid. This material was shown to be 87% pure, representing an 85% overall yield.

### EXAMPLE 4

### 2-Bromo-4-(chlorosulfonyl)benzoyl chloride

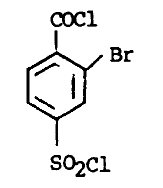

A 100 mL round bottom flask equipped with reflux condenser, temperature controller and magnetic stirrer was charged with 8.8 g (28 mmol) of the potassium salt of 2-bromo-4-sulfobenzoic acid, 21.5 mL (301 mmol) of thionyl chloride, and 0.12 g (1.7 mmol) of DMF. After heating for five hours, the cooled reaction mixture was filtered and evaporated to afford the desired product, 2-bromo-4-(chlorosulfonyl)benzoyl chloride.

### EXAMPLE 5

### 2-Bromo-4-(methylsulfonyl)benzoic Acid

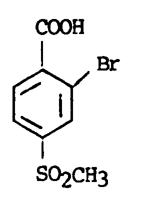

A 50 mL round bottom flask equipped with a reflux condenser, thermometer and magnetic stirrer was charged with 1.6 g (13 mmol) of sodium sulfite, 4.2 g (50 mmol) of sodium bicarbonate and 20 mL of water. The resulting slurry was heated to 75°C and 4.0 g (13 mmol) of 2-bromo-4-(chlorosulfonyl)benzoyl chloride was added over 30 minutes. After heating at 75°C for one hour, chloroacetic acid (1.8 g, 19 mmol) and 1.0 mL (19 mmol) of 50% aqueous sodium hydroxide were added sequentially to the aqueous solution of 2-bromo-4-sulfinylbenzoic acid, and the reaction mixture heated to reflux. Additional chloroacetic acid (2.0 g) was added as necessary to drive the reaction to completion. After heating for 16 hours, the reaction mixture was allowed to cool to ambient temperature and acidified with concentrated HCl. The precipitated solids were collected by filtration and dried to give 1.6 g of 2-bromo-4-(methylsulfonyl)benzoic acid, corresponding to a 46% yield.

### EXAMPLE 6

### 2-Nitro-4-(chlorosulfonyl)benzoyl Chloride

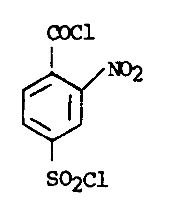

A 250 mL round bottom flask equipped with reflux condenser, thermometer, and magnetic stirrer was charged with 9.0 g (32 mmol) of the potassium salt of 2-nitro-4-sulfobenzoic acid, 10.7 mL (142 mmol) of thionyl chloride, 0.23 g (3.2 mmol) of DMF and 40 mL of toluene. After heating for one hour at 85°C, the cooled reaction mixture was filtered and evaporated to afford 8.2 g (92% yield) of 2-nitro-4-(chlorosulfonyl)benzoyl chloride as an amber oil.

### EXAMPLE 7

### 2-Nitro-4-(methylsulfonyl)benzoic Acid

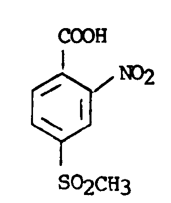

A 100 mL round bottom flask equipped with a reflux condenser, thermometer and magnetic stirrer was charged with 1.9 g (15 mmol) of sodium sulfite, 5.1 g (60 mmol) of sodium bicarbonate and 20 mL of water. The resulting slurry was cooled to 15°C and 4.0 g (14 mmol) of 2-nitro-4-(chlorosulfonyl)benzoyl chloride was added over 5 minutes. The reaction mixture was stirred at 15°C for three hours and then at ambient temperature overnight. After warming to 40°C, 3.1 g (27 mmol) of the sodium salt of chloroacetic acid was added to the aqueous solution of 2-nitro-4-sulfinylbenzoic acid and the reaction mixture heated to reflux. After heating for 7 hours, the reaction mixture was allowed to cool to ambient temperature, diluted with 30 mL of water and washed with 50 mL of ethyl acetate. The aqueous layer was acidified with concentrated HCl and extracted with 75 mL of ethyl acetate. The organic solution was concentrated to afford 3.0 (87% yield) of 2-nitro-4-(methylsulfonyl)benzoic acid as a pale yellow solid.

## Claims (Claims for the following Contracting State(s): AT, BE, CH, DE, FR, GB, IT, LI, LU, NL, SE)

1. An intermediate compound having the structural formula 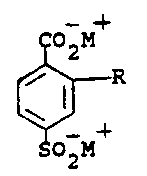 wherein R is chloro, bromo or nitro and M is hydrogen, sodium, potassium or ammonium.

2. The compound of Claim 1 wherein R is chloro and M is sodium.

3. The compound of Claim 1 wherein R is bromo and M is sodium.

4. The compound of Claim 1 wherein R is nitro and M is sodium.

5. A process for preparing 2-(chloro, bromo or nitro)-4-(alkylsulfonyl) benzoic acid compounds having the structural formula 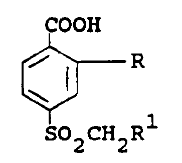 wherein R is chloro, bromo or nitro and R¹ is hydrogen or C₁-C₃ alkyl, comprising
1) reacting a compound having the structural formula 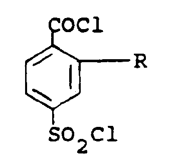 wherein R is as defined with a reducing agent along with a base in an aqueous medium to prepare a compound having the structural formula 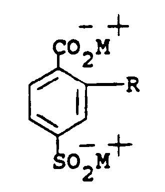 wherein R is chloro, bromo or nitro and M is sodium, potassium or ammonium and
2) reacting the prepared compound from step (1) with an α-halocarboxylic acid compound having the structural formula 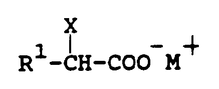 wherein X is chloro, bromo or iodo; R¹ is hydrogen or C₁-C₃ alkyl; and M is sodium, potassium or ammonium, to prepare a benzoic acid compound having the structural formula 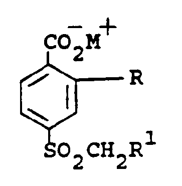 wherein M, R and R¹ are as defined, and finally acidifying the compound to prepare the desired product having the structural formula 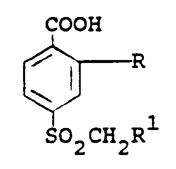 wherein R and R¹ are as defined.

6. The process of Claim 5 wherein R is chloro, R¹ is hydrogen, M is sodium and X is chloro.

7. The process of Claim 5 wherein R is nitro, R¹ is hydrogen, M is sodium and X is chloro.

8. The process of claim 5 wherein the α-halocarboxylic acid compound of step (2) is prepared in situ from about equal molar amounts of an α-halocarboxylic acid of the structural formula: 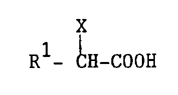 wherein R¹ and X are as defined and a base having sodium, potassium or ammonium as the cation.

9. The process of Claim 5 wherein the base is an inorganic base having a sodium, potassium or ammonium cation.

## Claims (Claims for the following Contracting State(s): ES)

1. A process for preparing 2-(chloro, bromo or nitro)-4-(alkylsulfonyl) benzoic acid compounds having the structural formula 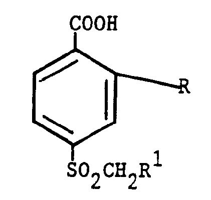 wherein R is chloro, bromo or nitro and R¹ is hydrogen or C₁₋₃ alkyl, comprising
1) reacting a compound having the structural formula 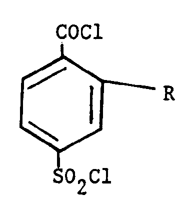 wherein R is as defined with a reducing agent along with a base in an aqueous medium to prepare a compound having the structural formula: 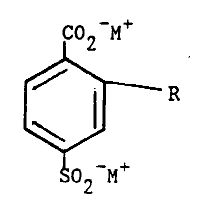 wherein R is chloro, bromo or nitro and M is sodium, potassium or ammonium and
2) reacting the prepared compound from step (1) with an α-halocarboxylic acid compound having the structural formula 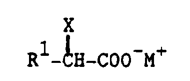 wherein X is chloro, bromo or iodo; R¹ is hydrogen or C₁₋₃ alkyl; and M is sodium, potassium or ammonium, to prepare a benzoic acid compound having the structural formula 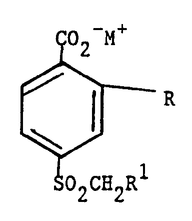 wherein M, R and R¹ are as defined, and finally acidifying the compound to prepare the desired product having the structural formula 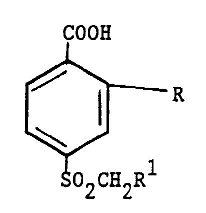 wherein R and R¹ are as defined.

2. The process of Claim 1 wherein R is chloro, R¹ is hydrogen, M is sodium and X is chloro.

3. The process of Claim 1 wherein R is nitro, R¹ is hydrogen, M is sodium and X is chloro.

4. The process of claim 1 wherein the α-halocarboxylic acid compound of step (2) is prepared in situ from about equal molar amounts of an α-halocarboxylic acid of the structural formula: 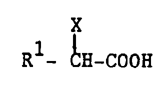 wherein R¹ and X are as defined and a base having sodium, potassium or ammonium as the cation.

5. The process of Claim 1 wherein the base is an inorganic base having a sodium, potassium or ammonium cation.

## Patentansprüche (Patentansprüche für folgende(n) Vertragsstaat(en): AT, BE, CH, DE, FR, GB, IT, LI, LU, NL, SE)

1. Zwischenprodukte mit der Strukturformel 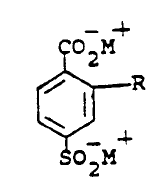 worin R für Chloro, Bromo oder Nitro und M für Wasserstoff, Natrium, Kalium oder Ammonium steht.

2. Die Verbindung von Anspruch 1, worin R für Chloro und M für Natrium steht.

3. Die Verbindung von Anspruch 1, worin R für Bromo und M für Natrium steht.

4. Die Verbindung von Anspruch 1, worin R für Nitro und M für Natrium steht.

5. Verfahren zur Herstellung von 2-(Chloro-, Bromo- oder Nitro)-4-(alkylsulfonyl)-benzoesäure-Verbindungen mit der Strukturformel 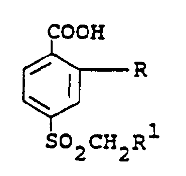 worin R für Chloro, Bromo oder Nitro und R¹ für Wasserstoff oder C₁₋₃-Alkyl steht, bei welchem
1) eine Verbindung mit der Strukturformel 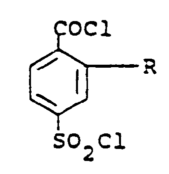 worin R die oben angegebene Bedeutung besitzt, mit einem Reduktionsmittel und mit einer Base in einem wäßrigen Medium umgesetzt wird, um eine Verbindung mit der Strukturformel 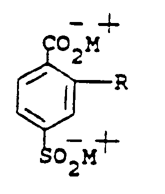 herzustellen, worin R für Chloro, Bromo oder Nitro und M für Natrium, Kalium oder Ammonium steht, und
2) die Verbindung aus Stufe (1) mit einer α-Halogenocarbonsäure-Verbindung mit der Strukturformel 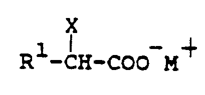 worin X für Chloro, Bromo oder Jodo, R¹ für Wasserstoff oder C₁₋₃-Alkyl und M für Natrium, Kalium oder Ammonium steht, umgesetzt wird, um eine Benzoesäure-Verbindung mit der Strukturformel 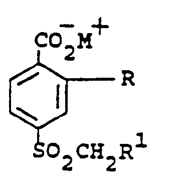 worin M, R und R¹ die oben angegebenen Bedeutungen besitzen, herzustellen, und abschließend die Verbindung angesäuert wird, um das gewünschte Produkt mit der Strukturformel 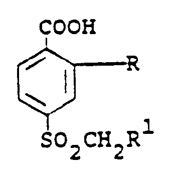 worin R und R¹ die angegebenen Bedeutungen besitzen, herzustellen.

6. Verfahren nach Anspruch 5, bei welchem R für Chloro, R¹ für Wasserstoff, M für Natrium und X für Chloro steht.

7. Verfahren nach Anspruch 5, bei welchem R für Nitro, R¹ für Wasserstoff, M für Natrium und X für Chloro steht.

8. Verfahren nach Anspruch 5, bei welchem die α-Halogenocarbonsäure-Verbindung der Stufe (2) in situ aus ungefähr äquimolaren Mengen einer α-Halogenocarbonsäure mit der Strukturformel 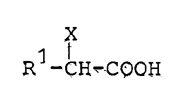 worin R¹ und X die oben angegebenen Bedeutungen besitzen, und einer Base, die Natrium, Kalium oder Ammonium als Kation aufweist, hergestellt wird.

9. Verfahren nach Anspruch 5, bei welchem die Base eine anorganische Base mit einem Natrium-, Kalium- oder Ammoniumkation ist.

## Patentansprüche (Patentansprüche für folgende(n) Vertragsstaat(en): ES)

1. Verfahren zur Herstellung von 2-(Chloro-, Bromo- oder Nitro)-4-(alkylsulfonyl)-benzoesäure-Verbindungen mit der Strukturformel 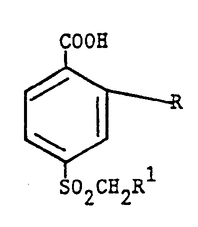 worin R für Chloro, Bromo oder Nitro und R¹ für Wasserstoff oder C₁₋₃-Alkyl steht, bei welchem
1) eine Verbindung mit der Strukturformel 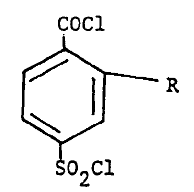 worin R die oben angegebene Bedeutung besitzt, mit einem Reduktionsmittel und mit einer Base in einem wäßrigen Medium umgesetzt wird, um eine Verbindung mit der Strukturformel 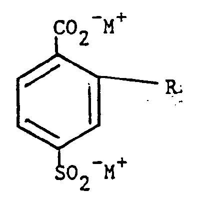 herzustellen, worin R für Chloro, Bromo oder Nitro und M für Natrium, Kalium oder Ammonium steht, und
2) die Verbindung aus Stufe (1) mit einer α-Halogenocarbonsäure-Verbindung mit der Strukturformel 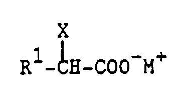 worin X für Chloro, Bromo oder Jodo, R¹ für Wasserstoff oder C₁₋₃-Alkyl und M für Natrium, Kalium oder Ammonium steht, umgesetzt wird, um eine Benzoesäure-Verbindung mit der Strukturformel 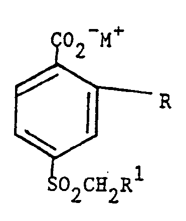 worin M, R und R¹ die oben angegebenen Bedeutungen besitzen, herzustellen, und abschließend die Verbindung angesäuert wird, um das gewünschte Produkt mit der Strukturformel 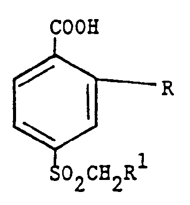 worin R und R¹ die angegebenen Bedeutungen besitzen, herzustellen.

2. Verfahren nach Anspruch 1, bei welchem R für Chloro, R¹ für Wasserstoff, M für Natrium und X für Chloro steht.

3. Verfahren nach Anspruch 1, bei welchem R für Nitro, R¹ für Wasserstoff, M für Natrium und X für Chloro steht.

4. Verfahren nach Anspruch 1, bei welchem die α-Halogenocarbonsäure-Verbindung der Stufe (2) in situ aus ungefähr äquimolaren Mengen einer α-Halogenocarbonsäure mit der Strukturformel 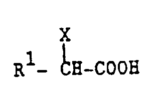 worin R¹ und X die oben angegebenen Bedeutungen besitzen, und einer Base, die Natrium, Kalium oder Ammonium als Kation aufweist, hergestellt wird.

5. Verfahren nach Anspruch 1, bei welchem die Base eine anorganische Base mit einem Natrium-, Kalium- oder Ammonium-Kation ist.

## Revendications (Revendications pour l'(les) Etat(s) contractant(s) suivant(s): AT, BE, CH, DE, FR, GB, IT, LI, LU, NL, SE)

1. Composé intermédiaire, répondant à la formule structurale 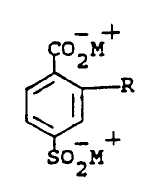 dans laquelle R est un radical chloro, bromo ou nitro et M est l'hydrogène, le sodium, le potassium ou l'ion ammonium.

2. Composé suivant la revendication 1, dans lequel R est le radical chloro et M est le sodium.

3. Composé suivant la revendication 1, dans lequel R est le radical bromo et M est le sodium.

4. Composé suivant la revendication 1, dans lequel R est le radical nitro et M est le sodium.

5. Procédé de production d'acides 2-(chloro, bromo ou nitro)-4-(alkylsulfonyl)benzoïques de formule structurale 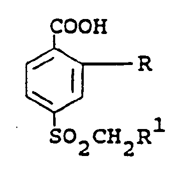 dans laquelle R est un radical chloro, bromo ou nitro et R¹ est l'hydrogène ou un groupe alkyle en C₁ à C₃, comprenant
1) la réaction d'un composé répondant à la formule structurale : 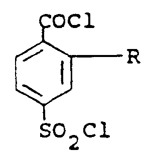 dans laquelle R est tel que défini, avec un agent réducteur conjointement avec une base en milieu aqueux pour préparer un composé répondant à la formule structurale 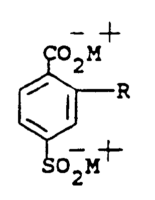 dans laquelle R est un radical chloro, bromo ou nitro et M est le sodium, le potassium ou l'ion ammonium et
2) la réaction du composé préparé dans l'étape (1) avec un composé d'acide α-halogénocarboxylique répondant à la formule structurale 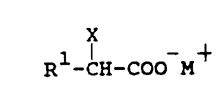 dans laquelle X est un radical chloro, bromo ou iodo ; R¹ est l'hydrogène ou un groupe alkyle en C₁ à C₃ ; et M est le sodium, le potassium ou l'ion ammonium, pour préparer un composé d'acide benzoïque répondant à la formule structurale 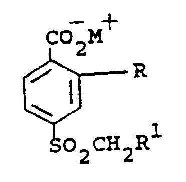 dans laquelle M, R et R¹ sont tels que définis, et finalement l'acidification du composé pour préparer le produit désiré répondant à la formule structurale 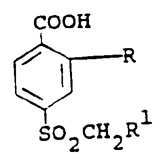 dans laquelle R et R¹ sont tels que définis.

6. Procédé suivant la revendication 5, dans lequel R est le radical chloro, R¹ est l'hydrogène, M est le sodium et X est le radical chloro.

7. Procédé suivant la revendication 5, dans lequel R est un groupe nitro, R¹ est l'hydrogène, M est le sodium et X est le radical chloro.

8. Procédé suivant la revendication 5, dans lequel le composé d'acide α-halogénocarboxylique de l'étape (2) est préparé in situ à partir de quantités à peu près équimolaires d'un acide α-halogénocarboxylique de formule structurale 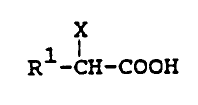 dans laquelle R¹ et X sont tels que définis et d'une base ayant comme cation le sodium, le potassium ou l'ion ammonium.

9. Procédé suivant la revendication 5, dans lequel la base est une base inorganique ayant un cation sodium, potassium ou ammonium.

## Revendications (Revendications pour l'(les) Etat(s) contractant(s) suivant(s): ES)

1. Procédé de production d'acides 2-(chloro, bromo ou nitro)-4-(alkylsulfonyl)benzoïques de formule structurale 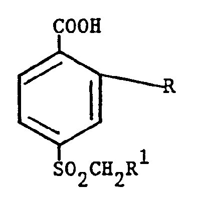 dans laquelle R est un radical chloro, bromo ou nitro et R¹ est l'hydrogène ou un groupe alkyle en C₁ à C₃, comprenant
1) la réaction d'un composé répondant à la formule structurale : 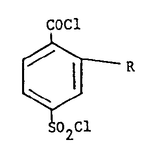 dans laquelle R est tel que défini, avec un agent réducteur conjointement avec une base en milieu aqueux pour préparer un composé répondant à la formule structurale 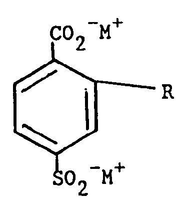 dans laquelle R est un radical chloro, bromo ou nitro et M est le sodium, le potassium ou l'ion ammonium et
2) la réaction du composé préparé dans l'étape (1) avec un composé d'acide α-halogénocarboxylique répondant à la formule structurale 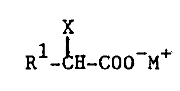 dans laquelle X est un radical chloro, bromo ou iodo ; R¹ est l'hydrogène ou un groupe alkyle en C₁ à C₃ ; et M est le sodium, le potassium ou l'ion ammonium, pour préparer un composé d'acide benzoique répondant à la formule structurale 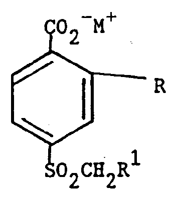 dans laquelle M, R et R¹ sont tels que définis, et finalement l'acidification du composé pour préparer le produit désiré répondant à la formule structurale 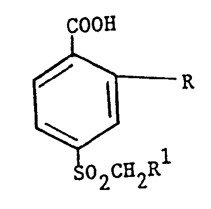 dans laquelle R et R¹ sont tels que définis.

2. Procédé suivant la revendication 1, dans lequel R est le radical chloro, R¹ est l'hydrogène, M est le sodium et X est le radical chloro.

3. Procédé suivant la revendication 1, dans lequel R est un groupe nitro, R¹ est l'hydrogène, M est le sodium et X est le radical chloro.

4. Procédé suivant la revendication 1, dans lequel le composé d'acide α-halogénocarboxylique de l'étape (2) est préparé in situ à partir de quantités à peu près équimolaires d'un acide α-halogénocarboxylique de formule structurale 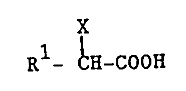 dans laquelle R¹ et X sont tels que définis et d'une base ayant comme cation le sodium, le potassium ou l'ion ammonium.

5. Procédé suivant la revendication 1, dans lequel la base est une base inorganique ayant un cation sodium, potassium ou ammonium.
